# EUROPÄISCHE PATENTSCHRIFT

(11) **EP 1 181 907 B1**
(45) Veröffentlichungstag und Bekanntmachung des Hinweises auf die Patenterteilung: **12.05.2004**
(21) Anmeldenummer: 01120339.5
(22) Anmeldetag: 24.08.2001
(51) Int. Cl.: A61F 2/18

(54) **Gehörknöchelchenprothese**
Auditory ossicles prosthesis
Prothèse d'osselets de l'oreille

(30) Priorität: 24.08.2000 DE 20014659 U
(43) Veröffentlichungstag der Anmeldung: 27.02.2002
(73) Patentinhaber: Heinz Kurz GmbH Medizintechnik, 72144 Dusslingen (DE)
(72) Erfinder: Kurz, Heinz, 72144 Dusslingen (DE)
(74) Vertreter: Möbus, Daniela, Dr.-Ing.

(56) Entgegenhaltungen:
- EP-A- 1 094 687
- DE-A- 19 744 789
- DE-A- 19 845 906
- DE-U- 29 904 770
- US-A- 4 601 723

## Beschreibung

Die Erfindung betrifft eine Gehörknöchelchenprothese wie in Anspruch 1 definiert mit einer zur Anlage an das Trommelfell vorgesehenen Kopfplatte, die über einen Schaft und ein Kugelgelenk mit einem am Steigbügel oder an der Steigbügelfußplatte anordenbaren Befestigungselement verbunden ist.

Da die relative Lage der Gehörknöchelchen zum Trommelfell bei jedem Menschen leicht differiert, muss bisher das Mittelohr vor Einsatz der Prothese genau vermessen und die Prothese insbesondere hinsichtlich der Lage der Kopfplatte und des Befestigungselements entsprechend den jeweiligen Gegegenheiten vor der Operation angepasst werden. Stellt sich bei der Operation heraus, dass die Prothese nicht angepasst ist, so ist die Möglichkeit einer In-Situ-Korrektur der Prothese wünschenswert. Außerdem kann es durch den Heilungsprozess nach der Operation zu einer geringfügigen Lageänderung des Trommelfells kommen, sodass die Kopfplatte nicht mehr richtig am Trommelfell anliegt, worunter die Schallübertragung leidet.

Aus der EP 1 094 687 A2 ist ein passives Hörsystem bekannt, das einen am Trommelfell anliegenden Kopf mit einer abgerundeten Oberfläche aufweist. An den Kopf schließt sich eine Koppelstange an, die mit einem Kopf einstückig verbunden sein kann und deren freies Ende über eine als Kugelgelenkkupplung ausgebildete Kupplung mit einem freien Ende des Koppelelements verbunden ist. Ein von der Kupplung abliegendes Ankoppelende des Koppelelements ist mit dem Steigbügelkopf gekoppelt.

Das EP-Dokument fällt unter Artikel 54(3) EPÜ und ist daher für die erfinderische Tätigkeit ohne Bedeutung.

Aus der DE 299 04 770 U1 ist ein als Teilprothese ausgebildetes Mittelohrimplantat mit einem flächigen Kopplungskörper zur Anlage an das Trommelfell bekannt. Mit dem Kopplungskörper ist ein länglicher Schaft verbunden. Der Schaft ist wiederum mit einem Fuß zur Anlage gegen den Steigbügel verbunden. Der Fuß weist eine sattelförmige Ausnehmung mit einer zentralen Aushöhlung zur Aufnahme eines Teils des Steigbügelbogens und des Steigbügelköpfchens auf.

Der vorliegenden Erfindung liegt daher die Aufgabe zu Grunde, eine Gehörknöchelchenprothese der eingangs genannten Art so weiterzubilden, dass die gelenkige Verbindung zwischen Kopfplatte und Befestigungselement einfach herstellbar ist.

Die Aufgabe wird mit einer Gehörknöchelchenprothese der vorstehend genannten Art erfindungsgemäß dadurch gelöst, dass das Kugelgelenk aus zwei Stegen gebildet ist, zwischen denen die Kugel in Ausnehmungen der Stege gelagert ist. Durch die gelenkige Verbindung lässt sich noch während der Operation sowohl die Lage der Kopfplatte als auch die Lage des Befestigungselementes exakt an den anatomischen Gegebenheiten im Mittelohr des Patienten ausrichten, sodass sichergestellt werden kann, dass die Kopfplatte einen optimalen Kontakt und Halt am Trommelfell und das Befestigungselement einen optimalen Sitz am Steigbügel bzw. der Steigbügelkopfplatte erhält. Sollten nach der Operation noch Veränderungen des Trommelfells durch Narbenzüge auftreten, so richtet sich die Kopfplatte automatisch parallel zum Trommelfell aus. Die Schallübertragung wird also durch den Heilungsprozess nicht mehr verschlechtert. Außerdem wird ein Drücken der Kanten der Kopfplatte auf das Trommelfell mit der Gefahr einer Extrusion sicher vermieden.

Für die gelenkige Verbindung von Kopfplatte und Befestigungselement gibt es verschiedene Möglichkeiten. So kann der Schaft beispielsweise gelenkig an der Kopfplatte angeordnet sein. Insbesondere bei Partialprothesen, bei denen das Befestigungselement am Steigbügel befestigt wird, ist diese Ausgestaltung von Vorteil, da bei einer gelenkigen Anordnung des Schaftes an der Kopfplatte auch sehr kurze Schaftlängen möglich sind. Sowohl fertigungstechnisch als auch von den Baumaßen der Prothese her ist es dabei am günstigsten, wenn der Schaft über ein in der Kopfplatte integriertes Gelenk mit der Kopfplatte verbunden ist. Das Gelenk sitzt dann geschützt innerhalb der Kopfplatte und nimmt kaum zusätzlichen Raum ein. Das Gelenk kann dabei ein Kugelgelenk sein, sodass eine Beweglichkeit der Kopfplatte gegenüber dem Schaft in allen Richtungen möglich ist. Der Schaft kann aber auch entweder zusätzlich oder alternativ zu einer gelenkigen Anordnung an der Kopfplatte gelenkig an dem Befestigungselement angeordnet sein. Auch hier ist es von Vorteil, wenn der Schaft über ein im Befestigungselement integriertes Gelenk, beispielsweise ein Kugelgelenk, mit dem Befestigungselement verbunden ist. Für das Gelenk wird weder zusätzliche Schaftlänge noch zusätzlicher Einbauraum benötigt, sofern es in dem Befestigungselement selbst integriert ist.

Insbesondere bei Vollprothesen, bei denen auch der Steigbügel ersetzt und somit das Befestigungselement an der Steigbügelfußplatte befestigt wird, kann auch der Schaft mindestens ein Gelenk, beispielsweise ein Kugelgelenk, aufweisen. Der Schaft solcher Prothesen ist ausreichend lang, um innerhalb von ihm mindestens ein Gelenk anzuordnen.

Weitere Vorteile ergeben sich, wenn die Kopfplatte rund oder oval ist und abgerundete Kanten aufweist. Die In-Situ-Justierung der Prothese bei dieser Ausgestaltung der Kopfplatte ist ausgesprochen sicher, da es dabei kaum zu Verletzungen des Trommelfells kommen kann. Die Kopfplatte kann auch Durchbrechungen aufweisen. Sind diese Durchbrechungen sehr groß, so erleichtern sie ebenfalls das Einsetzen der Prothese, da dann die Sicht auf den Steigbügel frei ist. Außerdem bewirken die Durchbrechungen ein gutes Anwachsen der Prothese am Trommelfell.

Für die Ausgestaltung des Befestigungselementes sind ebenfalls verschiedene Möglichkeiten gegeben. So kann das Befestigungselement als Clip oder glockenförmig oder stempelförmig ausgebildet sein.

Außerdem kann die Oberfläche der Gehörknöchelchenprothese an den Kontaktstellen zu Trommelfell und Steigbügel oder Steigbügelfußplatte aufgeraut sein, was sowohl ein Abrutschen während der Implantation verhindert als auch das Verwachsen mit dem Gewebe begünstigt.

Aufgrund der guten biokompatiblen Eigenschaften kann die Prothese vorzugsweise aus Titan hergestellt sein. Als ein besonders kostengünstiges und präzises Herstellungsverfahren kommt ein Laserschneiden / Laserschweißen der gesamten Prothese infrage.

Nachfolgend werden bevorzugte Ausführungsbeispiele von erfindungsgemäßen Gehörknöchelchenprothesen anhand der Zeichnungen näher beschrieben.

Es zeigen:
- Fig. 1: eine perspektivische Ansicht einer ersten Ausführungsform einer Prothese mit Gelenk in der Kopfplatte;
- Fig. 2: eine perspektivische Ansicht einer zweiten Ausführungsform einer Prothese mit Gelenk in der Kopfplatte;
- Fig. 3: eine dritte Ausführungsform einer Prothese mit Gelenk in der Kopfplatte;
- Fig. 4: eine erste Ausführungsform einer Prothese mit Gelenk im Schaft;
- Fig. 5: eine zweite Ausführungsform einer Prothese mit Gelenk im Schaft;
- Fig. 6: eine dritte Ausführungsform einer Prothese mit Gelenk im Schaft.

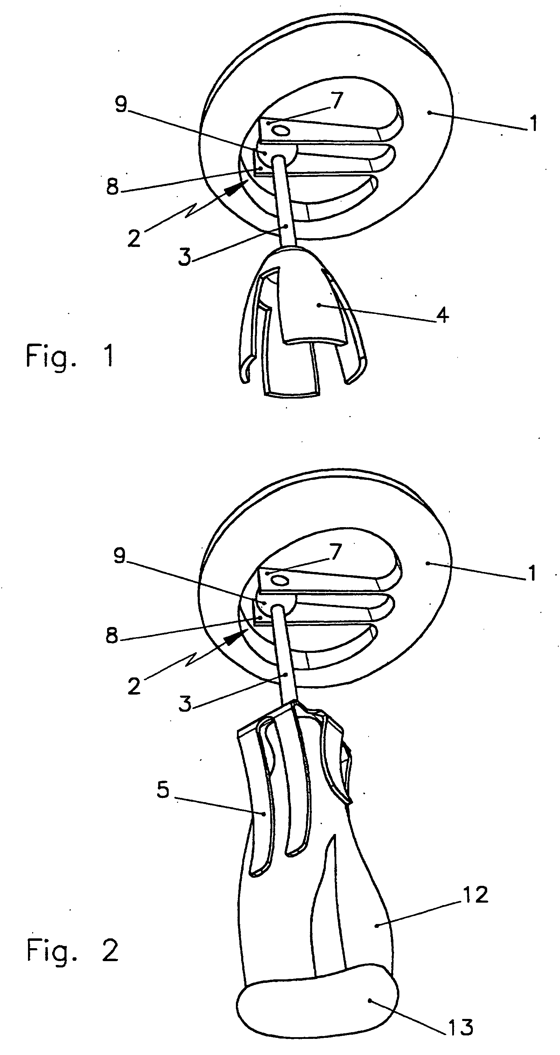

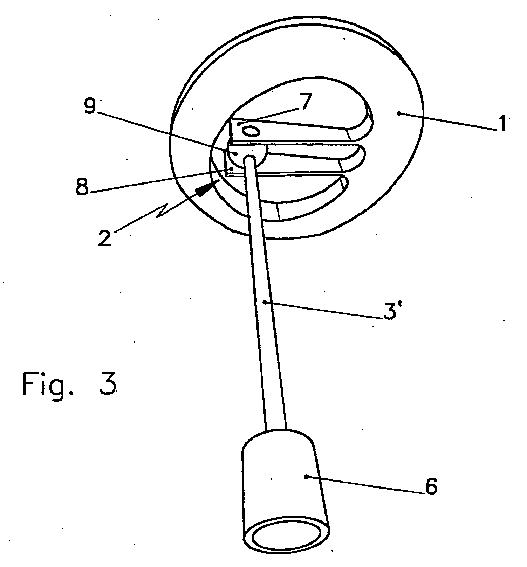

Bei den gezeigten sechs Beispielen von erfindungsgemäßen Prothesen sind jeweils identisch ausgebildete Teile mit denselben Bezugsziffern versehen. So weisen die Prothesen der Fig. 1 bis 3 alle dieselbe Kopfplatte 1 auf, die nach der Implantation gegen das Trommelfell anliegt. In die Kopfplatte 1 ist ein Kugelgelenk 2 integriert, an das sich im Falle der Prothesen aus den Fig. 1 und 2 ein kurzer Schaft 3 und im Falle der Prothese aus Fig. 3 ein längerer Schaft 3' anschließt. Am anderen Ende des Schaftes 3 bzw. 3' ist jeweils ein Befestigungselement angeordnet, im Fall der Fig. 1 ein glockenförmiges Befestigungselement 4, im Fall der Prothese aus Fig. 2 ein clipartiges, am Steigbügel 12 verankerbares Befestigungselement 5 und im Falle der Prothese aus Fig. 3 ein stempelförmiges Befestigungselement 6. Durch die Kugelgelenke 2 ist bei allen Prothesen eine gelenkige Verbindung zwischen der Kopfplatte 1 und den Befestigungselementen 4 bis 6 gegeben. Das Kugelgelenk 2 wird dabei von einer zwischen zwei im freien Innenbereich der Kopfplatte 1 angeordneten Stegen 7 und 8 in allen Richtungen frei beweglichen Kugel 9 gebildet.

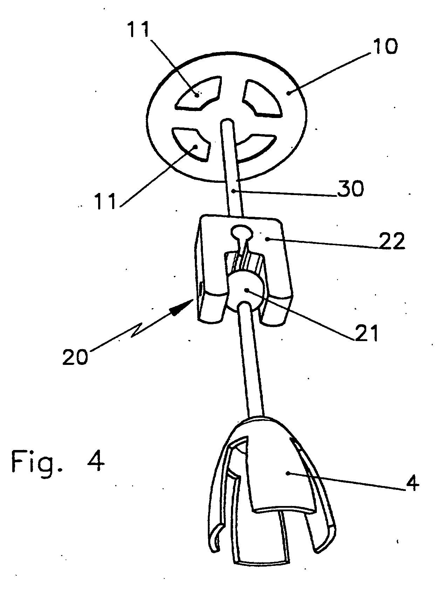

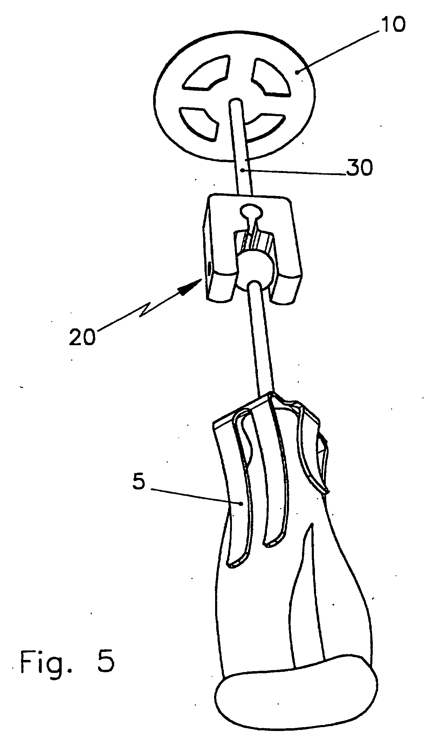

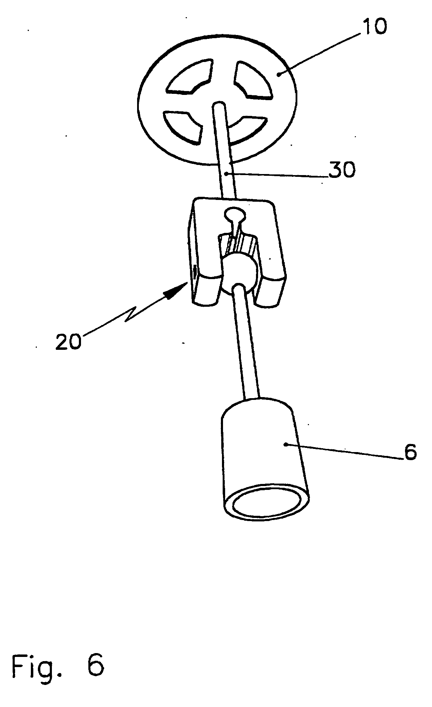

Die in den Fig. 4 bis 6 gezeigten Prothesen weisen gegenüber den Prothesen der Fig. 1 bis 3 etwas anders geformte Kopfplatten 10 mit mehreren Durchbrüchen 11 auf, an denen ein zweigeteilter Schaft 30 angeordnet ist. Die beiden Schaftteile sind durch ein Gelenk 20 miteinander verbunden. Auch das Gelenk 20 ist ein Kugelgelenk mit einer Kugel 21, die in einer klammerartigen Vorrichtung 22 in allen Drehrichtungen zumindest begrenzt beweglich gelagert ist. Am Fußende des Schaftes sind wieder die verschiedenen Befestigungselemente 4, 5 und 6 angeordnet.

Die Gelenkanordnung der Prothesen aus den Fig. 1 bis 3 eignet sich insbesondere für kurze Partialprothesen. Die in den Fig. 4 bis 6 gezeigten Lösungen mit Gelenken 20 im Schaft 30 benötigen eine bestimmte Mindestlänge des Schafts und sind daher insbesondere für Totalprothesen geeignet, bei denen der Schaft deswegen länger ist, weil auch der Steigbügel 12 in der Gehörknöchelchenkette mit ersetzt werden muss.

## Patentansprüche

1. Gehörknöchelchenprothese mit einer zur Anlage an das Trommelfell vorgesehenen Kopfplatte (1, 10), die über einen Schaft (3, 3', 30) und ein Kugelgelenk mit einem am Steigbügel (12) oder an der Steigbügelfußplatte (13) anordenbaren Befestigungselement (4, 5, 6) verbunden ist, **dadurch gekennzeichnet, dass** das Kugelgelenk aus zwei Stegen (7, 8) gebildet ist, zwischen denen die Kugel (9, 21) in Ausnehmungen der Stege (7, 8) gelagert ist.

2. Gehörknöchelchenprothese nach Anspruch 1, **dadurch gekennzeichnet, dass** der Schaft (3, 3') gelenkig an der Kopfplatte (1) angeordnet ist.

3. Gehörknöchelchenprothese nach Anspruch 2, **dadurch gekennzeichnet, dass** der Schaft (3, 3') über ein in der Kopfplatte (1) integriertes Gelenk (2) mit der Kopfplatte (1) verbunden ist.

4. Gehörknöchelchenprothese nach einem der Ansprüche 1 bis 3, **dadurch gekennzeichnet, dass** der Schaft (3, 3', 30) gelenkig an dem Befestigungselement (4, 5, 6) angeordnet ist.

5. Gehörknöchelchenprothese nach einem der Ansprüche 1 bis 4, **dadurch gekennzeichnet, dass** die Kopfplatte (1, 10) rund oder oval ist und abgerundete Kanten aufweist.

6. Gehörknöchelchenprothese nach einem der Ansprüche 1 bis 5, **dadurch gekennzeichnet, dass** die Kopfplatte (1, 10) Durchbrechungen (11) aufweist.

7. Gehörknöchelchenprothese nach einem der Ansprüche 1 bis 6, **dadurch gekennzeichnet, dass** das Befestigungselement (4, 5, 6) als Clip oder glockenförmig oder stempelförmig ausgebildet ist.

8. Gehörknöchelchenprothese nach einem der Ansprüche 1 bis 7, **dadurch gekennzeichnet, dass** ihre Oberfläche an den Kontaktstellen zu Trommelfell und Steigbügel (12) oder Steigbügelfußplatte (13) aufgeraut ist.

9. Gehörknöchelchenprothese nach einem der Ansprüche 1 bis 8, **dadurch gekennzeichnet, dass** sie durch Laserschneiden/Laserschweißen hergestellt ist.

10. Gehörknöchelchenprothese nach einem der Ansprüche 1 bis 9, **dadurch gekennzeichnet, dass** sie aus Titan gefertigt ist.

11. Gehörknöchelchenprothese nach einem der Ansprüche 1 bis 10, **dadurch gekennzeichnet, dass** die Ausnehmungen als Durchbrüche ausgebildet sind.

## Claims

1. Ossicular prosthesis comprising a head plate (1, 10) provided to bear against the eardrum and connected by means of a shaft (3, 3, 30) and a ball-and-socket joint to a fixing element (4, 5, 6) which can be arranged on the stirrup bone (12) or on the footplate (13) of the stirrup bone, **characterised in that** the ball-and-socket joint is formed of two bars (7, 8) between which the ball (9, 21) is supported in recesses in the bars (7, 8).

2. Ossicular prosthesis according to claim 1, **characterised in that** the shaft (3, 3) is hinged to the head plate (1).

3. Ossicular prosthesis according to claim 2, **characterised in that** the shaft (3, 3) is connected to the head plate (1) by means of a joint (2) integrated into the head plate (1).

4. Ossicular prosthesis according to one of claims 1 to 3, **characterised in that** the shaft (3, 3, 30) is hinged to the fixing element (4, 5, 6).

5. Ossicular prosthesis according to one of claims 1 to 4, **characterised in that** the head plate (1, 10) is round or oval and has rounded edges.

6. Ossicular prosthesis according to one of claims 1 to 5, **characterised in that** the head plate (1, 10) has openings (11).

7. Ossicular prosthesis according to one of claims 1 to 6, **characterised in that** the fixing element (4, 5, 6) is designed as a clip or is bell-shaped or punch-shaped.

8. Ossicular prosthesis according to one of claims 1 to 7, **characterised in that** its surface is roughened at the points where it contacts the eardrum and the stirrup bone (12) or the footplate (13) of the stirrup bone.

9. Ossicular prosthesis according to one of claims 1 to 8, **characterised in that** it is produced by laser cutting/laser welding.

10. Ossicular prosthesis according to one of claims 1 to 9, **characterised in that** it is made of titanium.

11. Ossicular prosthesis according to one of claims 1 to 10, **characterised in that** the recesses are designed as openings.

## Revendications

1. Prothèse pour osselet de l'oreille comportant une plaque de tête (1, 10) prévue pour s'appliquer contre le tympan, laquelle est reliée par une tige (3, 3', 30) et une articulation sphérique à un élément de fixation (4, 5, 6) pouvant être disposé sur l'étrier (12) ou sur la platine (13) de l'étrier, **caractérisée en ce que** l'articulation sphérique est constitué de deux pattes (7, 8), entre lesquelles la sphère (9, 21) est montée dans des évidements des pattes (7, 8).

2. Prothèse pour osselet de l'oreille selon la revendication 1, **caractérisée en ce que** la tige (3, 3') est disposée de manière articulée sur la plaque de tête (1).

3. Prothèse pour osselet de l'oreille selon la revendication 2, **caractérisée en ce que** la tige (3, 3') est reliée à la plaque de tête (1) par une articulation (2) intégrée dans la plaque de tête (1).

4. Prothèse pour osselet de l'oreille selon l'une des revendications 1 à 3, **caractérisée en ce que** la tige (3, 3', 30) est disposée de manière articulée sur l'élément de fixation (4, 5, 6).

5. Prothèse pour osselet de l'oreille selon l'une des revendications 1 à 4, **caractérisée en ce que** la plaque de tête (1, 10) est circulaire ou ovale et présente des bords arrondis.

6. Prothèse pour osselet de l'oreille selon l'une des revendications 1 à 5, **caractérisée en ce que** la plaque de tête (1, 10) présente des ajours (11).

7. Prothèse pour osselet de l'oreille selon l'une des revendications 1 à 6, **caractérisée en ce que** l'élément de fixation (4, 5, 6) est réalisé en tant que clip ou en forme de cloche ou en forme de poinçon.

8. Prothèse pour osselet de l'oreille selon l'une des revendications 1 à 7, **caractérisée en ce que** sa surface est rendue rugueuse aux points de contact avec le tympan et l'étrier (12) ou la platine (13) de l'étrier.

9. Prothèse pour osselet de l'oreille selon l'une des revendications 1 à 8, **caractérisée en ce qu'**elle est réalisée par des coupes au laser/soudage au laser.

10. Prothèse pour osselet de l'oreille selon l'une des revendications 1 à 9, **caractérisée en ce qu'**elle est fabriquée en titane.

11. Prothèse pour osselet de l'oreille selon l'une des revendications 1 à 10, **caractérisée en ce que** les évidements sont réalisés comme des ajours.
